# EUROPEAN PATENT APPLICATION

(11) **EP 1 273 316 A1**
(43) Date of publication of application: **08.01.2003**
(21) Application number: 01830442.8
(22) Date of filing: 02.07.2001
(51) Int. Cl.: A61M 5/32

(54) **Disposable safety syringe**

(71) Applicant: Restelli, Sergio, 00100 Rome (IT); Righi, Nardino, 20047 Brugherio (Milano) (IT); Rossi, Roberto, 20151 Milano (IT)
(72) Inventor: Restelli, Sergio, 00100 Rome (IT); Righi, Nardino, 20047 Brugherio (Milano) (IT); Rossi, Roberto, 20151 Milano (IT)
(74) Representative: Petruzziello, Aldo

(57) **Abstract**

A disposable syringe (100; 200) comprises:
- a syringe body (1) hollow on the inside and open at the front and the rear,
- a plunger (40) sliding inside the syringe body (1) with an injection stroke extending from a retracted syringe-filling position to a forward syringe-emptying position, said plunger (40) being provided at the rear with a shaft (50) which can be operated manually and brought out of the syringe body through the rear end thereof,
- an injection needle (10) integral with a needle carrier (11) attachable to the fore end (2) of the syringe body (1) and
- hooking means (60), operatively connected to the shaft (50) of the plunger, able to hook said needle-carrier (11) to pull it inside the syringe body,
The needle-carrier (11) comprising first engagement means able to engage with the fore end of the syringe body and second engagement means able to engage with the hooking means.

## Description

The present invention refers to a disposable safety syringe.

As is known, a syringe generally comprises a cylindrical body open at the rear to accommodate a plunger. A needle hollow on the inside is mounted at a head end of the syringe body. By retracting the plunger the liquid contained in a vial is drawn into the syringe body through the needle. By pressing on the plunger the liquid contained inside the syringe body is injected, by means of the needle, into the patient's body.

To comply with safety regulations and to avoid the transmission of infections diseases, syringes must generally be used just once and then discarded. For this reason there is growing demand on the market for disposable syringes able to prevent further use thereof.

Moreover, syringes generally have drawbacks from the point of view of safety. In fact, once the syringe has been used, the needle remains exposed at the head of the syringe body, with the risk of accidental injuries or needle sticks.

This drawback is overcome in part by European patent EP 0636381 which describes a protective device for syringe needles. In this case, when the plunger of the syringe reaches the end of its stroke, the fore end of the plunger shaft catches the needle. When the injection is completed the user must manually retract the shaft; in this manner the needle is pulled by the head of the shaft inside the syringe body in a safety position which avoids accidental needle sticks.

Said solution has problems during hooking the needle carrier and has the drawback that the user, having completed the injection, can forget to carry out retraction of the shaft, leaving the needle exposed and thus rendering the protective device ineffective.

Patent application PCT WO 99/37345 describes a disposable safety syringe which provides a needle-covering sleeve mounted axially on the syringe body and slidable from a retracted position, in which it leaves the needle exposed to allow injection, to an advanced position in which it completely covers the needle, preventing re-use of the syringe and acting as a protection against accidental needle sticks.

Once the injection has been carried out the sleeve is automatically brought into the deployed safety position, by means of an automatic mechanism and without any intervention by the user. However, said solution presents a certain complexity for provision and movement of the needle-covering sleeve.

The object of the present invention is to eliminate the drawbacks of the prior art, providing a disposable safety syringe that is practical, versatile, cheap and simple to make.

Another object of the present invention is to provide such a disposable syringe that is able to prevent further attempts at use.

Yet another object of the present invention is to provide such a disposable syringe that is extremely safe and able to prevent accidental injuries after use thereof.

These objects are achieved in accordance with the invention with the characteristics listed in appended independent claim 1.

Advantageous embodiments of the invention are apparent from the dependent claims.

The disposable syringe according to the invention comprises a syringe body hollow on the inside and open at the front and rear, a plunger that can slide inside the syringe body with an injection stroke extending from a retracted syringe-filling position to an advanced or forward syringe-emptying position, and an injection needle integral with a needle-carrier that can be engaged at the fore end of the syringe body. The plunger is provided at the rear with a shaft that can be operated manually and brought out of the syringe body through the rear end thereof.

The peculiarity of the disposable syringe according to the invention is represented by the fact that the needle-carrier has first engagement means able to engage in the fore end of the syringe body and second engagement means able to engage in hooking means operatively connected to the shaft of the plunger. In this manner, when the plunger reaches its end of stroke, the hooking means engage with the second engagement means of the needle-carrier and hook the needle-carrier pulling it inside the body of the syringe.

In a preferred embodiment the hooking means are a hooking device able to hook the needle-carrier. The hooking device is disposed slidably inside the piston shaft to be able to pass from a forward or advanced position, in which its fore part can engage with the needle-carrier to hook it, and a retracted position, in which the hooking device conveys the needle and the needle carrier inside the shaft, into a position of safety.

Elastic means are provided disposed between the shaft and the hooking device. The elastic means are loaded, when the hooking device is in its forward position, and release to push the hooking device from the advanced position to the retracted position.

Operating means are provided able to lock the hooking device in its forward position and release the hooking device to allow retraction thereof into the retracted position through the action of the elastic means.

The operating means can intervene automatically at the end of the injection, freeing the hooking device, or they can intervene through manual operation by the operator.

The advantages of the disposable syringe according to the invention are evident. In fact, once the injection has been completed, the hooking device hooks the needle-carrier and pulls it, together with the needle, inside the body of the shaft. In this manner the needle is in a safety position and thus accidental needle sticks and possible attempts to re-use the syringe are avoided.

Further characteristics of the invention will be made clearer by the detailed description that follows, referring to purely exemplary and therefore non-limiting embodiments thereof, illustrated in the appended drawings in which:
Figure 1 is an exploded, axonometric view, illustrating a first embodiment of the disposable safety syringe according to the invention;
Figure 2 is an axial sectional view of the shaft of the plunger of the syringe according to the invention;
Figure 3 is a cross sectional view taken along the plane of section III-III of Figure 2;
Figure 4 is an axial section of the needle-carrier hooking device of the syringe according to the invention;
Figure 5 is a perspective view illustrating the needle-carrier and the needle, partially cut off, of the syringe according to the invention;
Figure 6 is a part-sectional axial view of the syringe of Figure 1 assembled, in which the plunger is in a retracted position before carrying out the injection;
Figure 7 is a view like Figure 6, in which the plunger is in a forward or advanced position, in the vicinity of its forward end of stroke, at the end of the injection;
Figure 7a is an axial section, enlarged and partially broken off, taken along a plane of section at right angles to that of Figure 7, in which the plunger is in a forward position near the end of its forward end of stroke at the end of the injection;
Figure 8 is a view like Figure 6, in which the injection has been completed and the needle-carrier hooking device has hooked the needle-carrier pulling it inside the shaft of the plunger;
Figure 9 is an enlarged view of a detail of Figure 7 illustrating the head of the syringe body with the needle-carrier;
Figure 10 is an axial sectional view of the detail in Figure 9 along the plane of section X-X of Figure 9;
Figure 11 is a perspective, exploded view like Figure 1, illustrating a syringe according to a second embodiment of the invention;
Figure 12 is an axial sectional view, like Figure 7, of the syringe of Figure 11 according to a second embodiment of the invention;
Figure 13 is a rear view, from the right with reference to Figure 12, of the second embodiment of the syringe according to the invention.

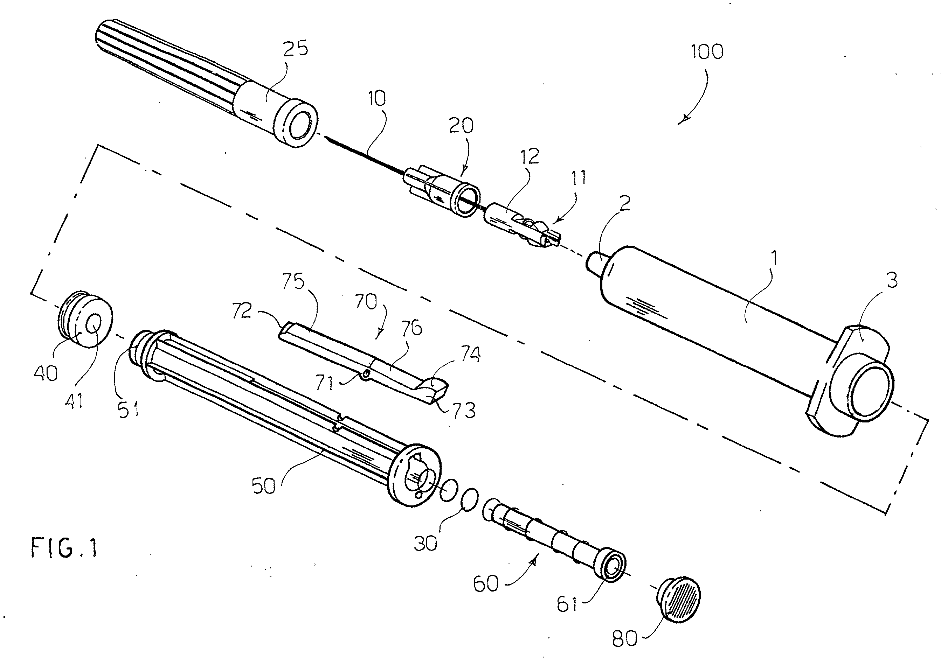

A first embodiment of the disposable safety syringe according to the invention, designated as a whole with reference numeral 100, is described with the aid of Figures 1-10.

For now with reference in particular to Figure 1, the syringe 100 comprises a cylindrical body 1, hollow on the inside, defining a cylindrical chamber. The rear end of the body 1 is open toward the outside and the body 1 has a flange 3 which protrudes radially outward.

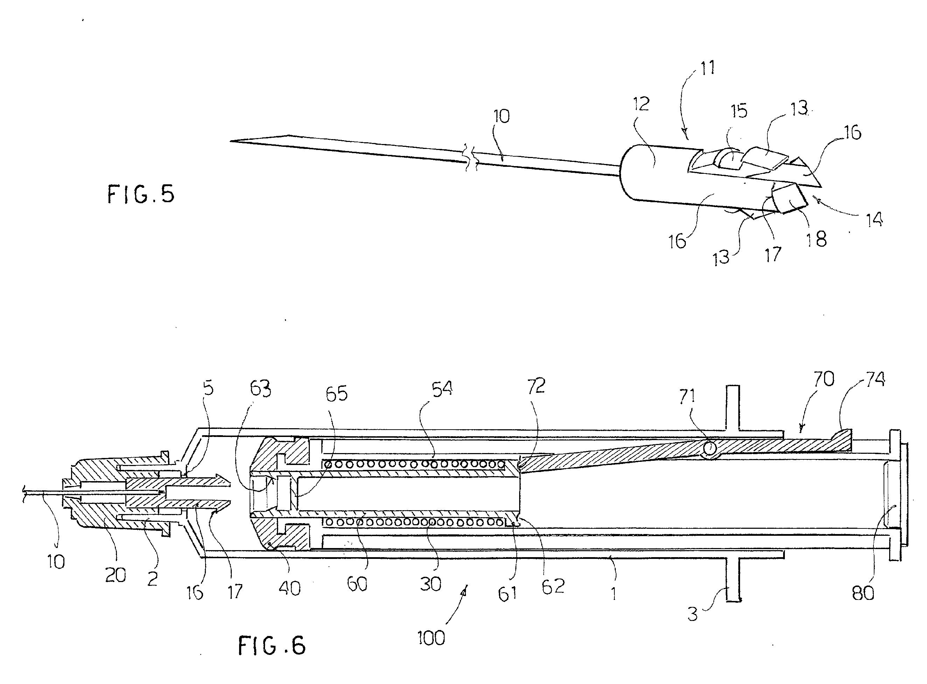

The fore end of the body 1 is tapered and ends with a head 2, open toward the outside, in the form of a cylindrical tang, with a smaller diameter than the body 1. As shown in Figures 6 to 10, a collar 5, which protrudes radially inward, is provided in the inner surface of the head 2.

A needle 10 is supported by a needle-carrier 11. As shown better in Figure 5, the needle-carrier 11 comprises a cylindrical block 12 which has an axial hole to receive one end of the needle 10. The cylindrical block 12 has four flexible tongues comprising a first pair of opposite tongues 13, and a second pair of opposite tongues 16. The tongues 13 and 16 are disposed around an axial hollow space 14 and are separated from each other by longitudinal slots of the hollow space 14. The two flexible tongues 13 of the first pair have a tapered, outwardly protruding outer end surface 19 which delimits an outward facing groove 15. The two flexible tongues 16 of the second pair have a tapered outwardly protruding outer end surface 18 which delimits an outward facing abutment surface 17. The tongues 13 of the first pair are slightly shorter than the tongues 16 of the second pair.

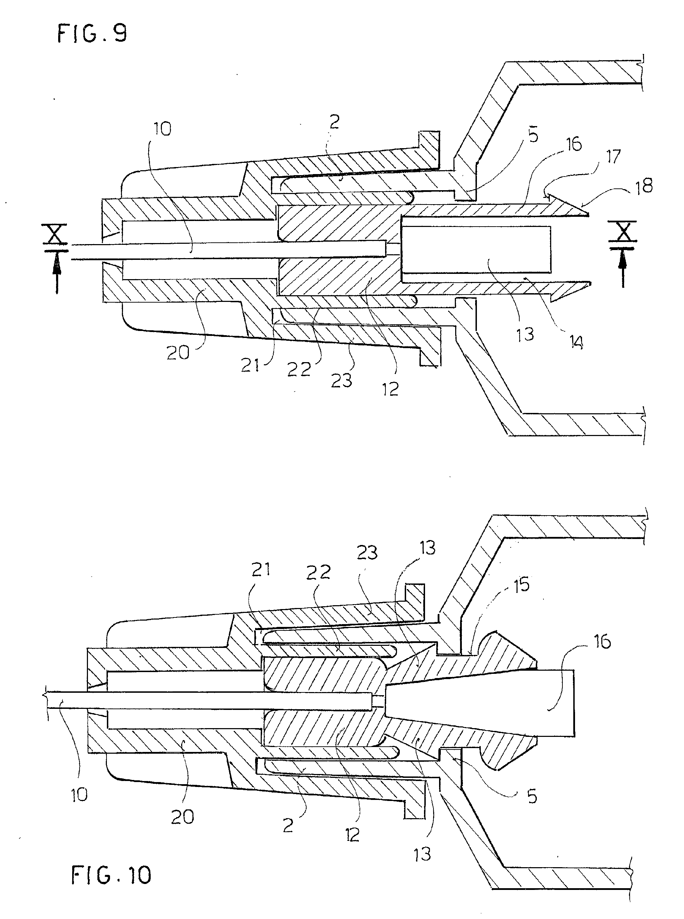

The needle-carrier 11 is inserted in the hole in the head 2 of the syringe and, as shown in Figure 10, the grooves 15 of the tongues 13 engage in the collar 5 of the head 2 so as to retain the needle-carrier. The tongues 16, on the other hand, remain free as shown in Figure 9. To keep the needle 10 axially aligned and to prevent it from being deployed, a retaining cap 20 having a frusto-conical shape is mounted on the head 2.

As shown better in Figures 9 and 10, the cap 20 provides an annular space or gap 21 defined by a cylindrical outer wall 23 and a cylindrical inner part 22. The head 2 of the syringe body is received in the space 21 and the cylindrical block 12 of the needle-carrier is centred inside the inner cylindrical part 22 of the cap, so as to keep the needle 10 in an axial position.

The needle 10 is covered by means of a needle guard 25 (Figure 1) which snap or screw engages in the cap 20.

The plunger 40 is made of plastic or rubber material and is of such a shape as to be able to slide with a tight seal inside the chamber of the syringe body 1. The plunger 40 has an axial through cavity 41 able to receive engageably a head 51 of a shaft or piston 50.

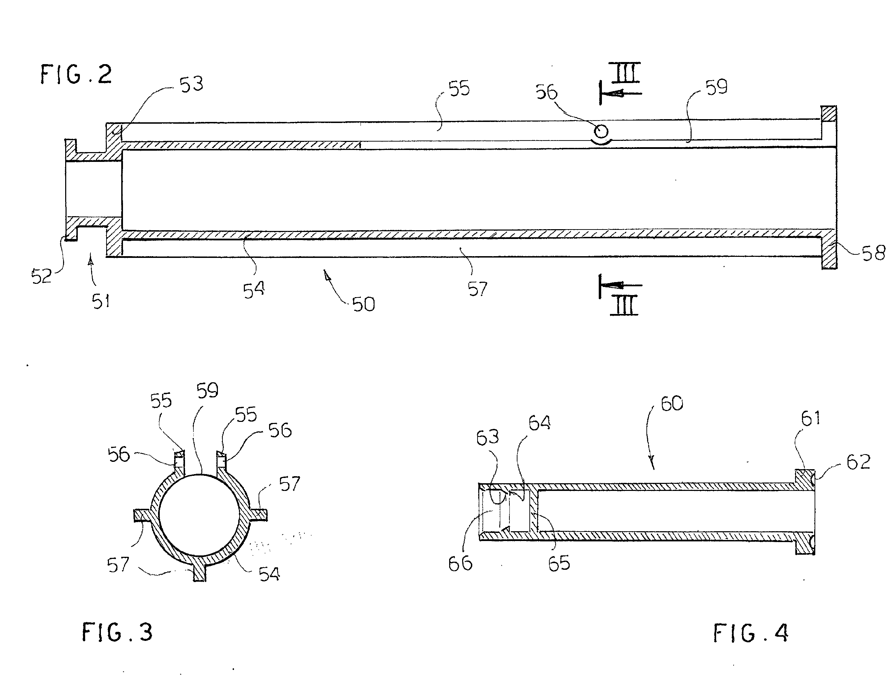

As shown in Figure 2, the shaft 50 has a cylindrical body hollow on the inside the head 51 of which has a first annular flange 52 which engages in an annular seat of the cavity 41 of the plunger and a second annular abutment flange 53 against which the base of the plunger 40 abuts.

As shown in Figure 3, three radial walls 57, spaced apart from each other by about 90°, and two non-radial walls 55, disposed parallel and slightly apart from each other, branch outward from the cylindrical body 54. The three radial walls 57 and the pair of parallel walls 55 extend substantially for the whole length of the shaft 50. Between the two parallel walls 55 the cylindrical body 54 has a longitudinal through slot 59 that extends from the rear end of the shaft 50 for more than half the length of the shaft 50.

Around halfway along the longitudinal slot 59 on each of the two parallel walls 55 a semicircular hole 56 is provided. The two holes 56 of the pair of parallel walls 55 are coaxial according to a transverse axis.

The rear end of the shaft 50 has a circular flange 58 able to receive a stopper 80 of the plunger shaft which forms a striking or abutment surface for operation of the shaft of the syringe by means of the operator's thumb.

With reference to Figures 1 and 4, a hooking device 60, able to hook the needle-carrier 11, has a cylindrical shape and has in its rear end a collar 61 having a larger diameter than the body of the hooking device 60. The outer diameter of the collar 61 is substantially equal to or slightly smaller than the inner diameter of the body 54 of the shaft 50 of the plunger. In this manner, the hooking device 60 can be inserted inside the body 50 of the shaft of the plunger and can slide on the inside thereof. The rear end of the collar 61 has an annular seat 62 in the form of an annular notch or recess.

In the fore part, the hooking device 60 has a hollow seat 66 delimited by a transverse wall 65 which separates the inside of the body of the hooking device 60. However, the inside of the body of the hooking device 60, beyond the transverse wall 65, could be made solid. The inner side wall 68 of the body of the hooking device 60 which delimits the seat 66 tapers slightly inward and forms an annular collar or ridge 63 which protrudes inward delimiting a retaining surface 64.

As shown in Figure 1, an operating lever 70 is formed by a rod with a rectangular section and has, in a substantially central position, a pivot 71 transversely disposed and having two ends protruding outward from the side walls of the lever 70. The pivot divides the lever 70 into two arms: a front arm 75 and a rear arm 76. The front arm 75 is slightly inclined with respect to the rear arm 76. The lever 70 has a slightly rounded front end part 72 and a larger rear part 73 having an outwardly protruding cam surface 74.

With reference to Figure 6, the ends of the pivot 71 of the lever 70 snap engage in the holes 56 of the parallel walls 55 of the plunger shaft 50. In this manner the lever 70 can turn around an axis of the pivot 71. In fact the front arm 75 of the lever 70 can go inside the body 54 of the plunger shaft, passing through the longitudinal slot 59 of the body of the plunger shaft.

The hooking device 60 is inserted inside the body 54 of the plunger shaft and a helicoidal spring 30 is disposed around the body of the hooking device 60. The ends of the spring 30 abut respectively on the abutment surface of the collar 53 of the fore part of the body 54 of the shaft of the plunger and on the abutment surface of the collar 61 of the rear part of the hooking device 60.

The spring 30 is loaded under compression and the hooking device 60 is locked, positioning the fore end 72 of the lever 70 in the annular seat 62 of the collar 61 of the rear part of the hooking device 60. In this situation the fore end of the hooking device 60 protrudes forward from the head 51 of the shaft 50 inside the seat 41 of the plunger 40. Lastly the shaft 50 of the plunger 40, comprising the hooking device 60 loaded by the spring 30 and locked by the lever 70, is inserted inside the body 1 of the syringe and the syringe 100 is thus ready for use.

Operation of the syringe 100 according to the invention will now be described.

In an initial situation the needle 10 is mounted on the head 2 of the syringe body 1, with the collar 5 of the head of the syringe body engaged in the grooves 15 of the tongues 13 of the needle carrier 11. The needle guard 25 is mounted on the cap 16 and keeps the needle 10 covered. The spring 30 is compressed inside the body 54 of the plunger shaft and around the body of the hooking device 60. The plunger 40 is mounted on the head of the shaft 50 and is inside the chamber of the syringe body 1.

Initially, the needle guard 25 is removed, the needle 10 is placed in the liquid to be aspirated, and the user retracts the shaft 50. The consequent retraction of the plunger 40 causes a vacuum in the chamber of the syringe body 1, thus the liquid, is aspirated through the needle 10 into the chamber of the syringe body 1 and the syringe is substantially in the configuration shown in Figure 6. Obviously the syringe could alternatively be pre-filled.

When the injection is carried out, the user presses the rear part 80 of the shaft 50 causing a forward movement of the plunger 40 which pushes the liquid which is injected through the needle 10.

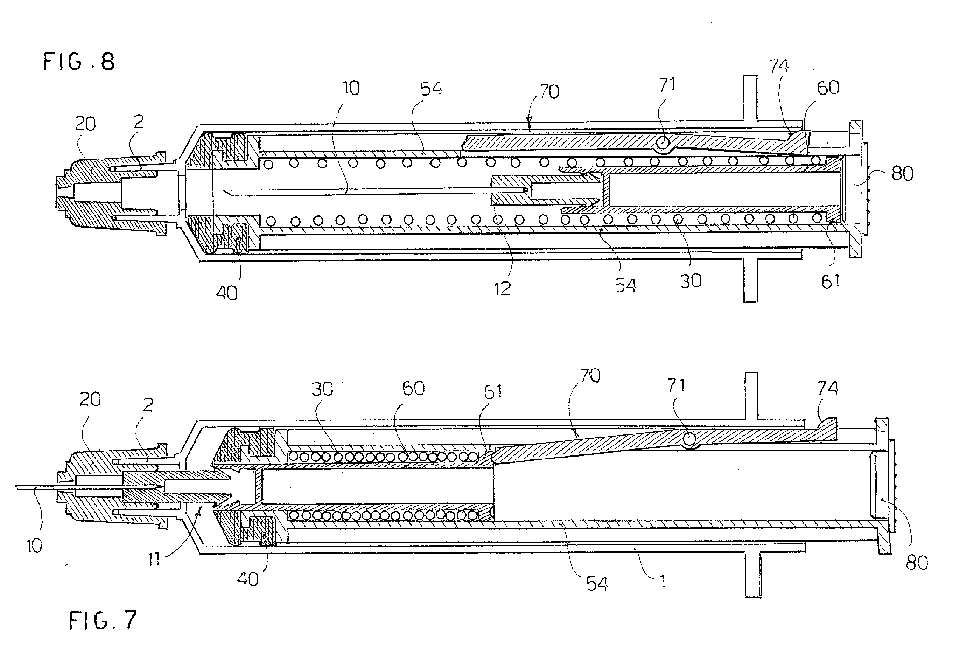

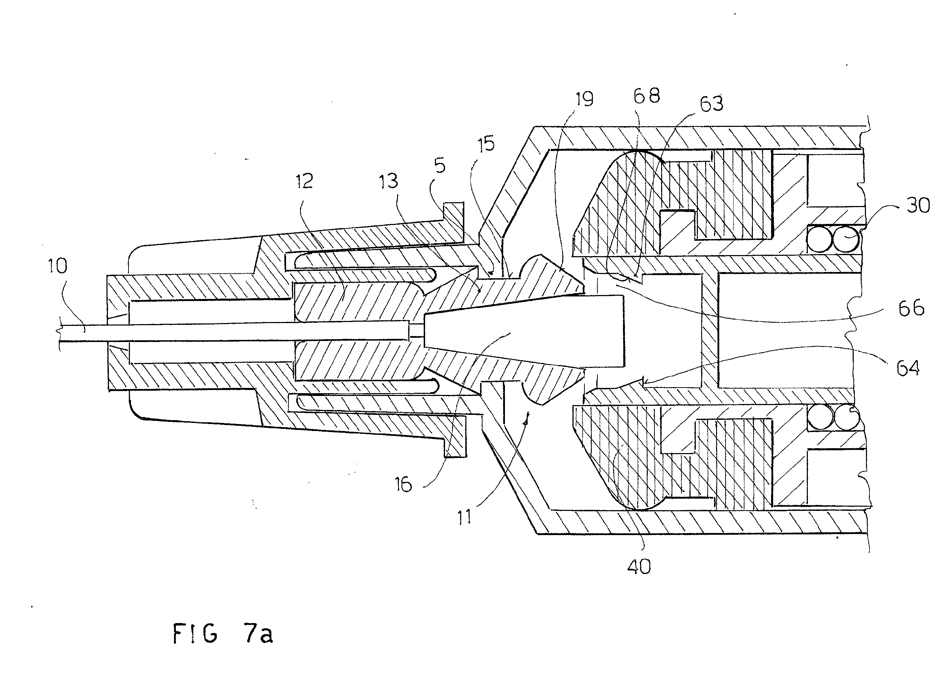

As shown in Figures 7 and 7a, when the plunger 40 arrives in the vicinity of the end point of its stroke, the tapered end parts 19 and 18, respectively, of the tongues 13 and 16 of the needle-carrier 11 enter the cavity 66 of the fore part of the hooking device 60 and enter into contact with the tapered inner surface 68 of the side part of the cavity 66.

As shown in Figure 7, the outer tapered surface 18 of the tongues 16 slides on the tapered inner surface 68 of the side wall of the fore part of the hooking device 60 and the tongues 16 bend inward. When the plunger 40 reaches the end point of its stroke, the collar 63 of the hooking device 60 passes the outer tapered surface 18 of the tongues 16. Consequently the tongues 16 snap outward and the abutment surface 17 of the tongues 16 engages with the retaining surface 64 of the hooking device 60. In this condition, the needle-carrier 11 is hooked by the fore part of the hooking device 60.

As shown in Figure 7a, when the plunger 40 is reaching the end point of its stroke, the tapered outer surface 19 of the tongues 13 slides on the tapered inner surface 68 of the side wall of the fore part of the hooking device. Consequently the tongues 13 bend inward and, when the plunger 40 has reached the end point of its stroke, the grooves 15 of the tongues 13 disengage from the collar 5 of the head of the syringe body. In this condition the needle-carrier 11 is hooked to the hooking device 60 and is no longer retained by the collar 5 of the syringe body.

When the plunger 40 is at the end of stroke, the cam surface of the rear end 73 of the lever 70 begins to encounter the rear end of the body 1 of the syringe. In this manner the cam surface 74 of the rear part of the lever pushes the end of the arm 76 of the lever toward the inside of the syringe and consequently the lever 70 performs a rotation (in a clockwise direction with reference to Figure 7) around the axis of the pivot 71 and the fore end 72 of the front arm 75 disengages from the seat 62 of the collar 61 of the hooking device 60, releasing the hooking device 60.

With reference to Figure 8, when the hooking device 60 is released by the lever 70, the spring 30 which was loaded under compression is released, expanding and causing retraction of the hooking device 60.

Since the needle-carrier is constrained to the fore part of the hooking device 60, when the spring 30 causes retraction of the hooking device 60, the needle 10 is pulled by the needle carrier inside the chamber of the shaft 50 of the plunger. In this manner the syringe cannot be used for a further injection and does not allow accidental needle sticks, since the needle remains protected inside the chamber of the shaft 50 of the plunger.

It should be noted that inside the syringe 100 the needle-carrier 11 is caught by the hooking device 60, when the plunger 40 reaches the end of stroke, at the end of the injection, and retraction of the hooking device inside the body 54 of the shaft of the piston takes place automatically by means of the operating lever 70, irrespective of the operator's will.

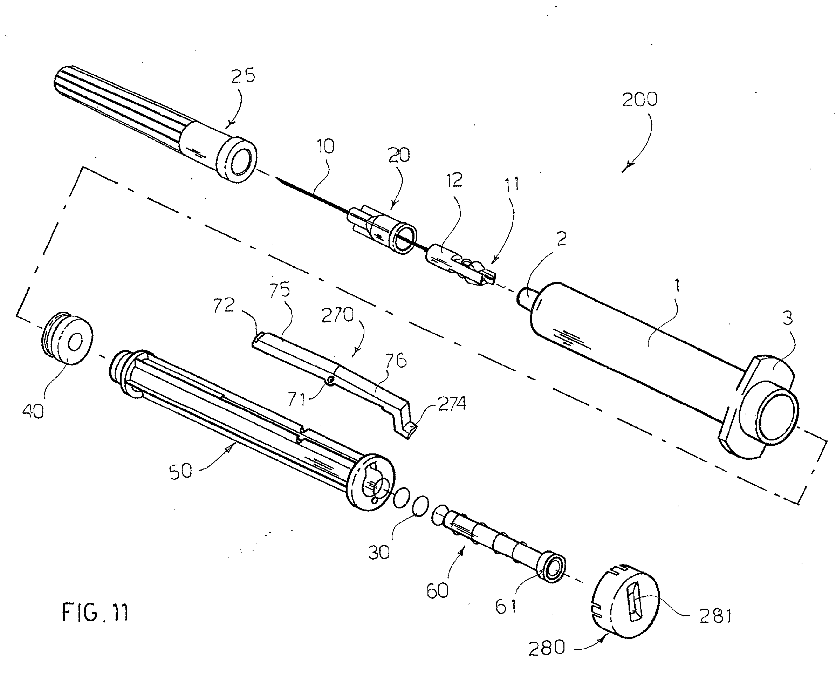

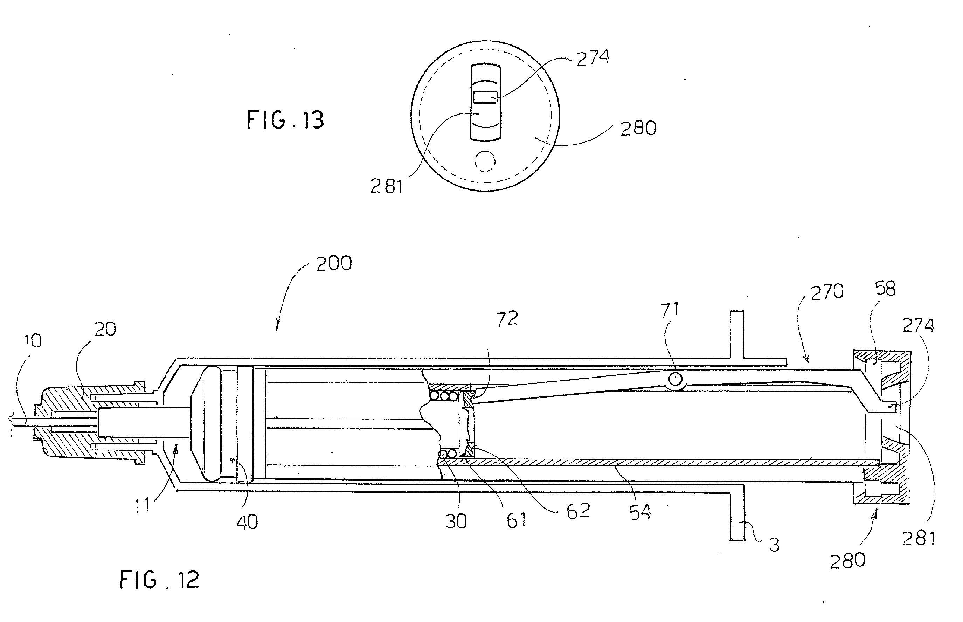

With reference to Figures 11-13 a syringe 200 according to a second embodiment of the invention is illustrated, in which like or corresponding elements to those already illustrated in the first embodiment are indicated with the same reference numerals and description thereof is omitted.

This second embodiment of the invention differs from the first embodiment only in the structure of the operating lever 270 and of the stopper 280 positioned at the rear end of the flange 58 of the plunger shaft. In fact the operating lever 270 has in place of the outwardly protruding cam surface 74 an inward protruding tooth 274 and the stopper 280 has a slot 281 inside which is disposed, with a certain play, the tooth 274 of the operating lever 270. In this manner, when the injection has been completed and the plunger has reached its end of stroke, the hooking device 60, after having hooked the needle-carrier 11, is not automatically released. In fact the lever 270 does not have the cam surface that abuts against the rear end of the body 1 of the syringe.

To obtain release of the hooking device 60, the operator must act manually on the operating lever 270 so that the end 72 of the lever disengages from the seat 62 of the hooking device. Said manual operation of the lever 270 can take place by pushing the tooth 274 of the lever 270 which protrudes outward from the slot 281 of the stopper 280.

In these two embodiments of the invention a hooking device 60 separate from the shaft 50 has been described. However, the hooking device 60 can be omitted and in its place the structure of the head of the hooking device 60 can be formed directly in the head 51 of the shaft 50. In this manner, when the plunger 40 reaches its end of stroke, the head 51 of the shaft directly catches the needle-carrier 11.

In this case, to automate the operation of re-entry of the piston shaft after it has caught the needle-carrier, spring means interposed between the shaft 50 of the plunger and the body 2 of the syringe can be provided. Said spring means are kept loaded under compression and are released when the plunger reaches the end of its stroke. When the spring means are released, they abut against a flange of the plunger shaft and cause retraction of the plunger which pulls with it the needle-carrier.

Numerous variations and modifications of detail within the reach of a person skilled in the art can be made to the present embodiments of the invention, without departing from the scope of the invention as set forth by the appended claims.

## Claims

1. A disposable syringe (100; 200) comprising:
- a syringe body (1) hollow on the inside and open at the front and rear,
- a plunger (40) sliding inside the syringe body (1) with an injection stroke extending from a retracted syringe-filling position to a forward syringe-emptying position, said plunger (40) being provided at the rear with a shaft (50) which can be manually operated and brought out of the syringe body through the rear end thereof,
- an injection needle (10) integral with a needle-carrier (11) attachable to the fore end (2) of the syringe body (11) and
- hooking means (60) operatively connected to said shaft (50) of the plunger, able to hook said needle-carrier (11) to pull it inside the syringe body;
**characterized in that**
said needle-carrier (11) comprises first engagement means able to engage to the front end of the syringe body and second engagement means able to engage with said hooking means (60).

2. A syringe according to claim 1, **characterized in that** said first engagement means of said needle-carrier are a first pair of opposed tongues (13) and said second engagement means of said needle-carrier are a second pair of opposed tongues (16).

3. A syringe according to claim 2, **characterized in that** said tongues (13, 16) protrude rearward and longitudinally from a cylindrical block (12) to which the needle (10) is fixed, said tongues being disposed around an axial hollow space (14) and being separated from each other by longitudinal slots, so as to be able to bend radially inward or outward.

4. A syringe according to claim 2 or 3, **characterized in that** each tongue (13) of said pair of tongues has a groove or notch (15) able to be engaged by a collar (5) provided in the inner surface of said head (2) and each tongue (16) of said second pair of tongues having an abutment surface (17) able to engage with a retaining surface (63) provided in said hooking means (60).

5. A syringe according to claim 4, **characterized in that**
- each of said tongues (13) of the first pair has at its end a tapered outer surface (19) which delimits said groove (15)
- each of said tongues (16) of the second pair has at its end a tapered outer surface (18) which delimits said abutment surface (17), and
- said hooking means have a cavity (66) with an inner tapered surface (68) which delimits said retention surface (64)
said tapered outer surface (19) of the tongues (13) able to slide on the inner tapered surface (68) of said hooking means (60) to cause inward bending of the said first pair of tongues (13) to release the groove (15) from the collar (5) of the syringe body, and said outer tapered surfaces (18) of the tongues (16) able to slide on the inner tapered surface (68) of said hooking means (60) to cause inward bending of the second pair of tongues (13) and to cause engagement of said abutment surface (17) of the tongues (16) with said retaining surface (64) of the hooking means (60).

6. A syringe according to any one of the preceding claims, **characterized in that** said hooking means comprise a hooking device (60) disposed slidably inside said shaft (50) to pass from a forward position in which its fore part can engage with said needle-carrier (11) to hook it, to a retracted position in which said hooking device conveys the needle-carrier and the needle inside said shaft.

7. A syringe according to claim 6, **characterized in that** it further comprises:
- elastic means (30) disposed between said shaft (50) and said hooking device (60), said elastic means being loaded when said hooking device is in its forward position; and
- operating means (70; 270) able to lock said hooking device in its forward position and release said hooking device to allow retraction thereof into the retracted position through the action of the elastic means.

8. A syringe according to claim 7, **characterized in that** said shaft (50) has an internally hollow cylindrical body (54) and said hooking device (60) has a substantially cylindrical body with a front seat (66) able to engage with said needle-carrier (11) to hook it and a rear collar (61) having a greater diameter than the body able to slide inside said hollow cylindrical body (54) of said shaft, said elastic means (30) being a helicoidal spring disposed around said hooking device (60), inside said cylindrical body (54) of said shaft and compressed between a collar (53) disposed at the front in said body of the shaft and said collar (61) disposed at the rear in said hooking device.

9. A syringe according to any one of claims 6 to 8, **characterized in that** said operating means (70) are a lever pivoted in said shaft (50) said lever having a first end (72) able to engage with said hooking device (60) to lock it and a second end (74; 174) able to be operated to cause release of said first end (72) from the hooking device.

10. A syringe according to claim 9, **characterized in that** said shaft (50) has longitudinal walls (56) protruding outward from said body (54) disposed substantially parallel to each other, each of said walls comprising a hole or seat for a pivot (56), said holes (56) being disposed coaxially in a transverse direction to receive the two ends of a pivot (72) disposed transversally in said lever (70), said pivot (72) dividing the lever (70) into a first arm (75) disposed at the front and a second arm (76) disposed a the rear, said first arm (75) being slightly oblique with respect to said second arm (76) and said body (54) of the shaft having a longitudinal slot (59) disposed between said two parallel walls (55) able to allow the passage of said first arm (75) of the lever (70).

11. A syringe according to claim 10, **characterized in that** said first arm (75) of said lever has a rounded end (72) able to engage in an annular seat (62) provided in the end of said collar (61) of the hooking device (60) to lock it in position.

12. A syringe (100) according to any one of claims 7 to 12, **characterized in that** said operating means (70) have automatic release means operated automatically to release said hooking device (60), when the plunger (40) reaches the end of stroke after having performed the injection, said automatic release means being an outward protruding cam surface (74), provided at the rear end of said second arm (76) of said lever (70), able to engage with the rear end of said body (1) of the syringe, when the plunger (40) reaches the end of stroke, generating a rotation of the lever (70) around the axis of the pivot (71) so that the front end (72) of the first arm (75) of the lever releases the hooking device (60).

13. A syringe (200) according to any one of claims 7 to 11, **characterized in that** said operating means (270) comprise manual release means, operated manually by the operator to release said hooking device (60), said manual release means being an inwardly protruding tooth (274), provided at the rear end of said second arm (76) of said lever (270), able to be operated manually by the operator to generate a rotation of the lever (270) around the axis of the pivot (71) so that the front end (72) of the first arm (75) of the lever releases the hooking device (60).

14. A syringe according to any one of claims 1 to 5, **characterized in that** said hooking means are formed integrally in the head (51) of said shaft (50) and comprise engagement means (63) able to engage with said second engagement means (16) of said needle-carrier.
